(19) Europäisches Patentamt
European Patent Office
Office européen des brevets

(11) **EP 3 602 040 B1**

(12) **EUROPÄISCHE PATENTSCHRIFT**

(45) Veröffentlichungstag und Bekanntmachung des Hinweises auf die Patenterteilung:
**20.07.2022 Patentblatt 2022/29**

(21) Anmeldenummer: **18706469.6**

(22) Anmeldetag: **15.02.2018**

(51) Internationale Patentklassifikation (IPC):
**G01N 33/22** *(2006.01)* **F02D 19/02** *(2006.01)*
**F02D 41/00** *(2006.01)*

(52) Gemeinsame Patentklassifikation (CPC):
**G01N 33/225; F02D 19/029; F02D 41/0027;**
F02D 2200/0602; F02D 2200/0606;
F02D 2200/0612; Y02T 10/30

(86) Internationale Anmeldenummer:
**PCT/EP2018/053826**

(87) Internationale Veröffentlichungsnummer:
**WO 2018/177651 (04.10.2018 Gazette 2018/40)**

(54) **VERFAHREN ZUM BESTIMMEN DER METHANZAHL EINES KOHLENWASSERSTOFFHALTIGEN BRENNGASGEMISCHS**

METHOD FOR DETERMINING THE METHANE INDEX OF A HYDROCARBON-CONTAINING COMBUSTION GAS MIXTURE

PROCÉDÉ DE DÉTERMINATION DE L'INDICE DE MÉTHANE D'UN MÉLANGE DE GAZ COMBUSTIBLES CONTENANT DES HYDROCARBURES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.03.2017 DE 102017106904**

(43) Veröffentlichungstag der Anmeldung:
**05.02.2020 Patentblatt 2020/06**

(73) Patentinhaber:
• **Endress+Hauser Flowtec AG**
 **4153 Reinach (CH)**
• **TrueDyne Sensors AG**
 **4153 Reinach (CH)**

(72) Erfinder:
• **HUBER, Christof**
 **3007 Bern (CH)**

• **REITH, Patrick**
 **79353 Bahlingen (DE)**
• **BADARLIS, Anastasios**
 **4127 Birsfelden (CH)**

(74) Vertreter: **Andres, Angelika Maria**
 **Endress+Hauser Group Services**
 **(Deutschland) AG+Co. KG**
 **Colmarer Straße 6**
 **79576 Weil am Rhein (DE)**

(56) Entgegenhaltungen:
EP-A1- 2 574 918 WO-A1-2015/075278
DE-A1-102014 115 566 DE-A1-102014 119 212
DE-A1-102015 117 468

**Beschreibung**

Die vorliegende Erfindung betrifft ein Verfahren zum Bestimmen der Methanzahl eines kohlenwasserstoffhaltigen Brenngasgemischs. Typische Brenngasgemische sind beispielsweise Erdgas oder Biogas.

Erdgas ist ein fossiler Energieträger. Es hat eine lagerstättenabhängige Zusammensetzung. Der Hauptbestandteil von Erdgas ist Methan, mit einem molaren Anteil von beispielsweise 75 % bis 99 %. Häufig enthält Erdgas auch größere Anteile an Ethan (1 % bis 15 %), Propan (1 % bis 10 %), Butan und Ethen. Weitere Nebenbestandteile sind Schwefelwasserstoff, Stickstoff, Kohlenstoffdioxid und Wasserdampf.

Biogas ist ein brennbares Gasgemisch unterschiedlicher Zusammensetzung, das durch Vergärung von Biomasse jeder Art entsteht. Es enthält im Rohzustand insbesondere Methan (bis zu 60%) und Kohlenstoffdioxid als Hauptkomponenten. Weiterhin sind Stickstoff, Sauerstoff, Schwefelwasserstoff, Wasserdampf und Ammoniak enthalten. Schwefelwasserstoff und Ammoniak müssen vor dem Verbrennen bzw. vor dem Einspeisen ins Erdgasnetz entfernt werden.

Die Veröffentlichung DE 10 2014 115566 A1 offenbart ein Messgerät mit einem integralen Messkanal zur Leitung eines Messmediums, mit einem Sensor, zur Bestimmung einer ersten thermisch-physikalische Eigenschaft, ausgesucht aus der Wärmeleitfähigkeit, der Temperaturleitfähigkeit bzw. der spezifischen Wärmekapazität des Messmediums, und einem vibronischen Sensor zur Bestimmung der Viskosität oder der Dichte des Messmediums.

Die Veröffentlichung DE 10 2014 119212 A1 offenbart eine Messanordnung zum Messen der Dichte von fließfähigen Medien mit einer Pumpe zum Treiben eines definierten Volumenstroms eines Mediums in einem Fluidpfad, mit einer Differenzdruckmessanordnung zum Erfassen eines Druckabfalls aufgrund des Volumenstroms des Mediums in dem Fluidpfad, mit einem vibronischen Dichtemesser welcher mindestens ein schwingfähiges Messrohr, zum Führen des Mediums aufweist eine Auswertungsvorrichtung, welche dazu eingerichtet ist, anhand des Volumenstroms, des volumenstromabhängigen Druckabfalls und der Schwingfrequenz des Oszillators die Dichte des Mediums zu ermitteln.

Die Veröffentlichung WO 2015 075278 A1 offenbart ein Verfahren zum Charakterisieren eines brennbaren Gases in einer Vorrichtung mit einem Heizelement zwischen zwei Temperatursensoren, sowie einer Drossel zwischen zwei Drucksensoren, wobei auf Basis einer Druckdifferenz bei einem Volumenstrom und zwei Temperaturen bei einer gegebenen Heizleistung ein thermophysikalischer Parameter, wie der Brennwert, der Wobbe-Index oder die Methanzahl bestimmt wird.

Die Veröffentlichung EP 2 574 918 A1 offenbart ein Verfahren und einen Sensor zur Bestimmung physikalischer Gaseigenschaften, wie der Dichte p, Wärmekapazität cp, des Massen- oder Volumenflusses oder einer Kombination dieser Größen von Gasgemischen zur Korrelation brenntechnisch relevanter Größen dieser Gasgemische mittels Kombination eines ersten Sensors für die physikalische Gaseigenschaft $\gamma$ in Bezug auf das Verhältnis zwischen Fließgeschwindigkeit und Wärmediffusivität der Gasgemische.

Die noch unveröffentlichte Patentanmeldung DE 102015117468.5 offenbart ein Verfahren zum Verfahren zum Bestimmen des Wobbe-Indexes bzw. des Brennwerts sowie des Inertgasanteils von Gasgemischen, welche insbesondere Erdgas oder Biogas aufweisen, auf der Basis der Viskosität und der Dichte bzw, Schallgeschwindigkeit.

Das Gasnetz dient jedoch mit steigender Tendenz als Energiespeicher für alternativ erzeugtes Gas aus "Power to Gas" ($H_2$) und "Biogas" ($CH_4 + CO_2$) welches mit Flüssiggas ($C_2H_6+C_3H_8$) angereichert wird. Damit verändert sich die Gaszusammensetzung im Netz signifikant. Die Gasqualität beim Verbraucher schwankt stark und es können schnelle Änderungen auftreten. Der Wasserstoffanteil kann bis zu 20% betragen. Der Wobbe-Index ist als Maß für eine gute Brennerregelung nur noch bedingt geeignet, da sich $H_2$ anders verhält als Erdgas.

Die noch unveröffentlichte Patentanmeldung DE 102016121226.1 offenbart ein Verfahren zum Bestimmen des Brennwerts von einem wasserstoffhaltigen Brenngasgemischs.

Wenn ein Brenngasgemisch zum Betreiben eines Verbrennungsmotors eingesetzt werden soll kommt es neben dem Brennwert auf die Klopffestigkeit des Brenngasgemischs an. Die Methanzahl gibt eine Beschreibung des Klopfverhaltens eines Brenngasgemischs in einem Verbrennungsmotor, wobei ein Brenngasgemisch mit einer Methanzahl von Null die gleiche Klopffestigkeit aufweist wie reiner Wasserstoff und ein mit einer Methanzahl von 100 die gleiche Klopffestigkeit wie reines Methan. Einzelheiten zur Methanzahl sind beispielsweise in "BHKW und Methanzahl, Einfluss der Gasbeschaffenheit auf den Motorbetrieb" der Arbeitsgemeinschaft für sparsamen und umweltfreundlichen Energieverbrauch oder in "Algorithm for methane number determination for natural gasses", ISBN 87-7795-125-5 von Paw Andersen beschrieben.

Die Methanzahl MZ' eines Brenngasgemischs kann demnach experimentell mit einem Prüfmotor oder als gewichteter Mittelwert der Methanzahlen $MZ_i$ von Komponentengruppen des Brenngasgemischs berechnet werden.

$$MZ' = \frac{1}{100} \sum y_i MZ_i$$

Das obige Berechnungsverfahren setzt jedoch die Kenntnis der Methanzahlen $MZ_i$ der Komponentengruppen und deren Anteil $y_i$ am Brenngasgemisch voraus. Zudem dürfen sich die Methanzahlen der Komponentengruppen nicht zu stark voneinander unterscheiden. Anderenfalls sind die Komponentengruppen neu zu fassen. Dies erfordert ein langwieriges Verfahren mit aufwändigen Messungen zum Bestimmen der Anteile der Komponentengruppen, insbesondere dann, wenn dieses auf eine Bestimmung der Anteile der einzelnen Komponenten hinausläuft.

Es ist daher die Aufgabe der vorliegenden Erfindung ein einfacheres Verfahren zum Bestimmen der Methanzahl eines kohlenwasserstoffhaltigen Brenngasgemischs anzugeben. Die Aufgabe wird erfindungsgemäß gelöst durch das Verfahren gemäß dem unabhängigen Patentanspruch 1.

Das erfindungsgemäße Verfahren zum Bestimmen der Methanzahl eines kohlenwasserstoffhaltigen Brenngasgemischs, welches insbesondere Erdgas oder Biogas aufweist, umfasst:

Strömen Lassen des Brenngasgemischs durch eine Messanordnung;

Bestimmen eines ersten Messwerts einer von der Viskosität des strömenden Brenngasgemischs abhängigen ersten Messgröße,

Bestimmen eines zweiten Messwerts einer von der Dichte des strömenden Brenngasgemischs zweiten Messgröße;

Bestimmen eines zum ersten Messwert und zweiten Messwert zugehörigen Druckmesswerts des strömenden Brenngasgemischs, und

Bestimmen eines zum ersten Messwert und zweiten Messwert zugehörigen Temperaturmesswerts des strömenden Brenngasgemischs; und

Bestimmen der Methanzahl als Funktion des ersten Messwerts, des zweiten Messwerts, des Druckmesswerts und des Temperaturmesswert.

Das erfindungsgemäße Verfahren ermöglicht damit eine einfache und robuste Bestimmung der Methanzahl auf Basis weniger Messgrößen ohne die genauen Anteile einzelner Komponenten bzw. Komponentengruppen genau kennen zu müssen. Die unterschiedlichen Korrelationen von Dichte und Viskosität mit den Anteilen der für die Methanzahl bestimmenden Komponentengruppen ermöglichen, als Zwischenschritt eine hinreichend genaue Bestimmung der mittleren molaren Masse von Kohlenwasserstoffverbindungen sowie den Anteil von Fremdkomponenten, wie im Folgenden näher erläutert ist. Aus diesen Größen lässt sich über Korrelationsrechnung die Methanzahl bestimmen. Wenngleich die Vorgehensweise über die beschriebenen Zwischenschritte intuitiver nachzuvollziehen ist, da die Methanzahl den genannten stofflichen Eigenschaften des Brenngasgemischs zugeordnet wird, kann auf die Schritte verzichtet werden, und die Methanzahl kann auf Basis von Korrelationsrechnungen direkt den beobachteten viskositäts- und dichteabhängigen Messgrößen zugeordnet werden.

Die Messwerte von Druck und Temperatur dienen insbesondere dazu, deren Einflüsse auf den ersten Messwert der ersten Messgröße und den zweiten Messwert der zweiten Messgröße zu korrigieren, beispielsweise durch Rückführen des ersten Messwerts und des zweiten Messwerts auf Referenzbedingungen, insbesondere Standardbedingungen.

In einer Weiterbildung der Erfindung charakterisiert die erste Messgröße eine Dämpfung von Schwingungen eines dem Brenngasgemisch ausgesetzten Oszillators, oder sie beschreibt einen Druckabfall über einer Drossel. Die erste Messgröße kann beispielsweise eine Resonanzbreite einer erzwungenen Schwingung, ein Zusammenhang zwischen Phasenwinkel zwischen einem Anregungssignal und einem Schwingungssignal des Oszillators einerseits und dem Verhältnis zwischen Anregungsfrequenz und Eigenfrequenz des Oszillators andererseits, sowie das Verhältnis zwischen Schwingungsamplitude und Anregungssignalamplitude sein.

In einer Weiterbildung der Erfindung umfasst die zweite Messgröße eine Eigenfrequenz eines dem Brenngasgemisch ausgesetzten Oszillators oder die Schallgeschwindigkeit des Brenngasgemischs.

In einer Weiterbildung der Erfindung umfasst das erfindungsgemäße Verfahren weiterhin die Bestimmung eines die Wärmeleitfähigkeit des Brenngasgemischs charakterisierenden dritten Messwerts, wobei der Dritte Messwert ebenfalls in die Ermittlung der Methanzahl eingeht. Der dritte Messwert dient insbesondere zur Bestimmung des Wasserstoffanteils in dem Brenngasgemisch.

In einer Weiterbildung des erfindungsgemäßen Verfahrens zum Bestimmen der Methanzahl eines kohlenwasserstoffhaltigen Brenngasgemisch, welches insbesondere Erdgas oder Biogas aufweist, umfasst es die folgenden Schritte:

Strömen Lassen des Brenngasgemischs durch eine Messanordnung;

Bestimmen Viskositätsmesswerts des strömenden Brenngasgemischs;

Bestimmen eines Dichte- oder Schallgeschwindigkeitsmesswerts des strömenden Brenngasgemischs;

Bestimmen eines Werts für die mittlere molare Masse der in dem Brenngasgemisch enthaltenen Kohlenwasserstoffe als Funktion zumindest des Viskositätsmesswerts und des Dichte- oder Schallgeschwindigkeitsmesswerts;

Bestimmen der Methanzahl als Funktion des Werts für die mittlere molaren Masse der in dem Brenngasgemisch enthaltenen Kohlenwasserstoffverbindungen.

In einer Weiterbildung der Erfindung umfasst das Bestimmen des Werts für die mittlere molare Masse der in dem Brenngasgemisch enthaltenen Kohlenwasserstoffverbindungen:

Ermitteln eines Werts für die mittlere molare Masse des strömenden Brenngasgemischs in Abhängigkeit vom Dichte- oder Schallgeschwindigkeitsmesswert des strömenden Brenngasgemischs; und

Ermitteln eines Anteils mindestens einer Fremdkomponente an dem Brenngasgemisch wobei die Fremdkomponente frei von Kohlenwasserstoffverbindungen ist;

Ermitteln des Werts für die mittlere molare Masse der in dem Brenngasgemisch enthaltenen Kohlenwasserstoffverbindungen als die mittlere molare Masse des um die mindestens eine Fremdkomponente bereinigten Brenngasgemischs, in Abhängigkeit des ermittelten Werts für die mittlere molare Masse des strömenden Brenngasgemischs und des Anteils der mindestens einen Fremdkomponente an dem strömenden Brenngasgemisch.

In einer Weiterbildung der Erfindung umfasst die mindestens eine Fremdkomponente in dem Brenngasgemisch enthaltene Inertgase, insbesondere Kohlenstoffdioxyd und Stickstoff.

In einer Weiterbildung der Erfindung umfasst das Bestimmen des Inertgasanteils an dem strömenden Brenngasgemisch die folgenden Schritte:

Bestimmen eines ersten Werts einer den Energiegehalt des Brenngasgemischs charakterisierenden Größe in Abhängigkeit vom Viskositätsmesswert;

Bestimmen eines zweiten Werts der den Energiegehalt des Brenngasgemischs charakterisierenden Größe in Abhängigkeit vom Dichte- oder Schallgeschwindigkeitsmesswert des strömenden Brenngasgemischs und unabhängig vom Viskositätsmesswert; und

Bestimmen des Werts für den Inertgasanteil des strömenden Brenngasgemischs in Abhängigkeit von einer Abweichung zwischen dem ersten Wert und dem zweiten Wert der den Energiegehalt des Brenngasgemischs charakterisierenden Größe.

In einer Weiterbildung der Erfindung ist die den Energiegehalt charakterisierende zweite Größe der Wobbe Index oder der Brennwert des strömenden Brenngasgemischs.

In einer Weiterbildung der Erfindung umfasst die mindestens eine Fremdkomponente den in dem Brenngasgemisch enthaltenen molekularen Wasserstoff.

In einer Weiterbildung der Erfindung wird der Anteil des molekularen Wasserstoffs auf Basis einer Messung der Wärmeleitfähigkeit des Brenngasgemischs bestimmt.

In einer Ausgestaltung der Erfindung wird der Wasserstoffanteil $X_{H2}$ als Funktion der Standardwärmeleitfähigkeit der molaren Masse bzw. der Dichte und der (Standard)Viskosität berechnet.

In einer Weiterbildung der Erfindung wird der Anteil des molekularen Wasserstoffs an dem strömenden Brenngasgemisch bestimmt,

wobei die mittlere molare Masse des um den molekularen Wasserstoff bereinigten Brenngasgemischs in Abhängigkeit des ermittelten Werts für die mittlere molare Masse des strömenden Brenngasgemischs und des Anteils des molekularen Wasserstoffs an dem strömenden Brenngasgemisch bestimmt wird,

wobei der Inertgasanteil an dem um den molekularen Wasserstoff bereinigten Brenngasgemisch bestimmt wird,

wobei die mittlere molare Masse der in dem Brenngasgemisch enthaltenen Kohlenwasserstoffverbindungen in Abhängigkeit von der mittleren molaren Masse des um den molekularen Wasserstoff bereinigten Brenngasgemischs in Abhängigkeit des ermittelten Werts für die mittlere molare Masse des um den molekularen Wasserstoff bereinigten Brenngasgemischs und des Inertgasanteils an dem um den molekularen Wasserstoff bereinigten Brenngasgemisch bestimmt wird; und

wobei die Methanzahl des Brenngasgemischs enthaltenen Kohlenwasserstoffverbindungen anhand des ermittelten Werts für die mittlere molare Masse der in dem Brenngasgemisch enthaltenen Kohlenwasserstoffverbindungen bestimmt wird.

In einer Weiterbildung der Erfindung wird die Methanzahl des strömenden Brenngasgemischs auf Basis des ermittelten Werts für die Methanzahl an den in dem Brenngasgemisch enthaltenen Kohlenwasserstoffverbindungen bestimmt.

Die ermittelten Werte der Methanzahl werden gemäß einer Weiterbildung der Erfindung zur Steuerung eines Verbrennungsmotors, einer Mischvorrichtung für Brenngasgemische oder einer sonstigen Datenverarbeitungseinrichtung zur Verfügung gestellt.

Das Erfindungsgemäße Verfahren beruht insbesondere auf der statistischen Analyse der physikalischen Eigenschaften von mehreren tausend Brenngasgemischen in Abhängigkeit von ihrer Zusammensetzung. Die Zusammensetzung wurde mittels Gaschromatographie bestimmt. Für die ermittelten Zusammensetzungen wurden die physikalischen Eigenschaften der Gasgemische bei verschiedenen Druck- und Temperaturwerten rechnerisch ermittelt. Gleichermaßen wurden die physikalischen Eigenschaften einiger reiner Gase berechnet. Für die rechnerische Ermittlung der physikalischen Eigenschaften wurde ein Programm des NIST verwendet, nämlich "Reference Fluid Thermodynamic and Transport Properties Database", kurz REFPROP, Version 9.1, welches unter der Adresse www.nist.gov/srd/nist23.cf, zugänglich ist.

Für die Berechnung der Wärmeleitfähigkeit für beliebige Brenngaszusammensetzungen eignet sich insbesondere die PPDS (physical property data software) welche von NEL, einem Unternehmen von TÜV Süd angeboten wird. Informationen hierzu sind unter www.tuvnel.com/site2/subpage/software_solutions_ppds erhältlich.

Methanzahlen für Brenngasgemische sind mit einer Software von EON nach der sogenannten AVL-Methode zu berechnen. Einzelheiten hierzu sind unter www.eon.com/en/business-areas/technical-services/gascalc-software/gascalc-module.htm zu erfahren.

Eine experimentelle Bestimmung der physikalischen Größen ist gleichermaßen möglich, bedeutet aber einen größeren Aufwand.

Zu den rechnerisch ermittelbaren physikalischen Größen gehören:

- Dichte: $\rho(T,p)$ mit NISTrefprop oder PPDS
- Molare Masse mit NISTrefprop oder PPDS
- Schallgeschwindigkeit SOS mit NISTrefprop oder PPDS
- Dynamische Viscosität: $\eta(T,p)$ mit NISTrefprop oder PPDS
- Brennwert: CV mit NISTrefprop oder PPDS
- Wobbe index: $WI = CV/\sqrt{SG}$ mit NISTrefprop oder PPDS
- Wärmeleitfähigkeit $\lambda(T,p)$ mit PPDS
- Methanzahl mit www.eon.com/en/business-areas/technical-services/gascalcsoftware/gascalc-module.html.

Auf Basis der obigen Daten wurde das erfindungsgemäße Verfahren entwickelt.

Die erfindungsgemäße Vorrichtung dient zum Bestimmen von zumindest der Methanzahl eines kohlenwasserstoffhaltigen Gasgemisches mit dem erfindungsgemäßen Verfahren, wobei die Vorrichtung umfasst:

eine von dem Gasgemisch durchströmbare Messanordnung, mit einem Temperatursensor, einem Drucksensor und einem vibronischen Sensor zum Bestimmen eines Viskositätsmesswerts und ggf. eines Dichtemesswerts des strömenden Gasgemischs; und

eine Auswertungseinheit zur Durchführung von verfahrensgemäßen Rechenschritten, insbesondere zur Berechnung von Eigenschaften des strömenden Gasgemischs, insbesondere der Methanzahl, wobei die Auswertungseinheit dazu eingerichtet ist, das erfindungsgemäße Verfahren durchzuführen.

In einer Weiterbildung der Erfindung ist der vibronische Sensor ein MEMS-Sensor, welcher mindestens ein durchströmbares, schwingfähiges Messrohr und/oder mindestens einen vom strömenden Gasgemisch umgebenen Oszillator, insbesondere in Form mindestens eines schwingfähigen Kragträgers oder einer schwingfähigen Stimmgabel aufweist.

In einer Weiterbildung der Erfindung umfasst die Vorrichtung weiterhin einen Wärmeleitfähigkeitssensor.

Die Erfindung wird nun anhand der in den Zeichnungen dargestellten Ausführungsbeispiele näher erläutert. Es zeigt:

Fig. 1: Ein Flussdiagramm für ein erstes Ausführungsbeispiel des erfindungsgemäßen Verfahrens;

Fig. 2: Ein Flussdiagramm für ein zweites Ausführungsbeispiel des erfindungsgemäßen Verfahrens; und

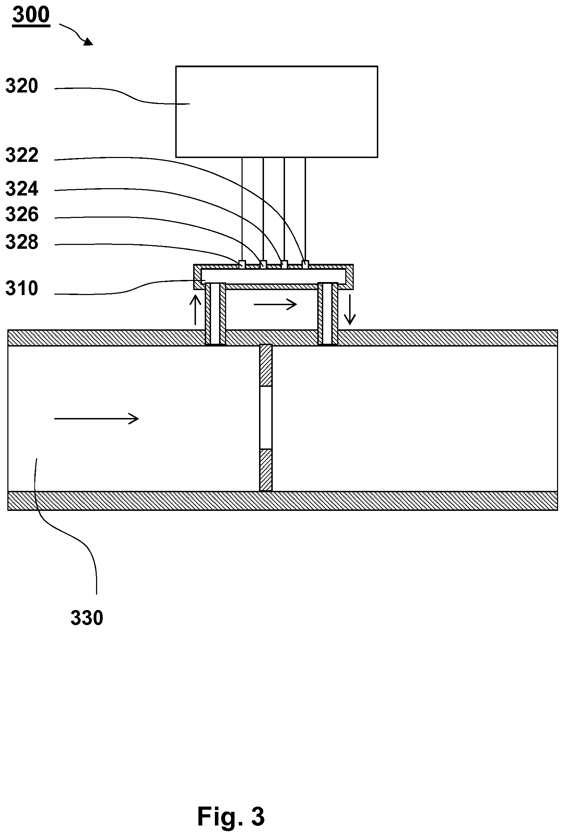

Fig. 3: Eine schematische Darstellung eines Ausführungsbeispiel für eine erfindungsgemäße Vorrichtung zum Bestimmen der Methanzahl.

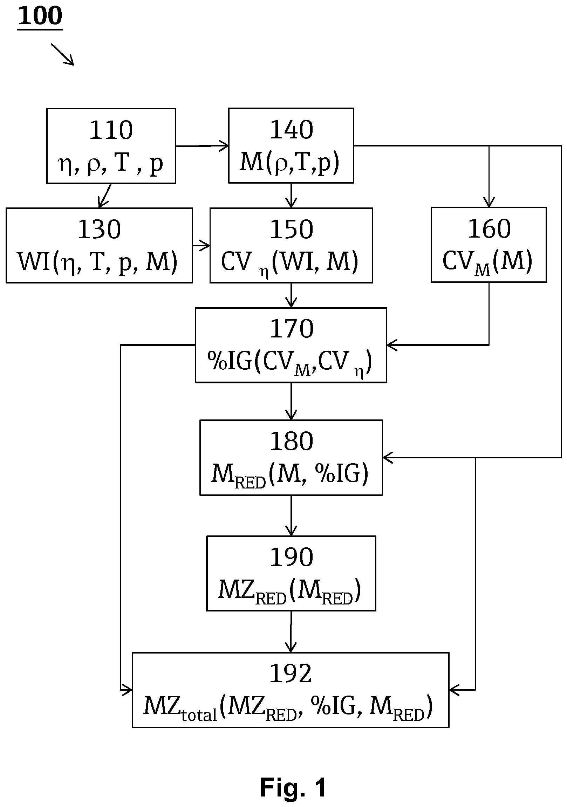

Das in Fig. 1 dargestellte erste Ausführungsbeispiel des erfindungsgemäßen Verfahrens ist insbesondere für Brenngasgemische geeignet, die nur geringe Mengen an Wasserstoff enthalten. Das Verfahren 100 umfasst in einem Schritt 110 das Erfassen eines Viskositätsmesswerts $\eta$, eines Dichtemesswerts p, eines Temperaturmesswerts T und eines Druckmesswerts p des strömenden Brenngasgemischs, wobei die genannten Messwerte möglichst gleichzeitig zu erfassen sind und die dazu erforderlichen Sensoren vorzugsweise möglichst nahe beieinander angeordnet sind, so dass die Messwerte ein Wertetupel des Gasgemisches in einem thermodynamischen Zustand bilden. Die Messung der Viskosität erfolgt beispielsweise mit einem vibronischen Sensor, insbesondere einem schwingenden Cantilever, der von dem Gasgemisch umgeben ist. Optional kann aus dem aktuellen Viskositätsmesswert bei einem gegebenen Druck p und einer gegebenen Temperatur T zunächst ein Viskositätsmesswert bei Standardbedingungen bestimmt werden. Die Dichte des Brenngasgemischs kann ebenfalls mit dem vibronischen Sensor bestimmt werden, da dessen Resonanzfrequenz von der Dichte abhängt.

Ein geeigneter mikromechanischer vibronischer Sensor ist beispielsweise in der noch unveröffentlichten deutschen Patentanmeldung DE 10 2016 124 910.6 beschrieben.

In einem Schritt 130 wird auf Basis eines Viskositätsmesswerts, sei es der direkt gemessene Viskositätsmesswert oder ein daraus abgeleiteter Viskositätsmesswert bei Referenzbedingungen, ein erster Wert für den Wobbe-Index WI($\eta$, T, p) des Brenngasgemischs bestimmt, wobei dieser erste Wert für den Wobbe-Index unabhängig von der Dichte des Brenngasgemischs bestimmt wird.

Es ist jedoch vorteilhaft, zunächst aus einem aktuellen Viskositätswert $\eta$(T,p) bei einem gegebenen Druck p und einer gegebenen Temperatur T zunächst eine Standardviskosität bei Standardbedingungen $\eta_{ref}$ zu bestimmen, wobei dann der Wobbe Index auf Basis der Standardviskosität bei Standardbedingungen zu berechnen ist. Die Standardviskosität $\eta_{ref}$ ist aus einem aktuellen Viskositätswert beispielsweise zu berechnen mit einem Polynom in $\eta$, p und T, insbesondere:

$$\eta_{ref} = C_0 + C_1 \cdot \eta + C_2 \cdot p + C_3 \cdot T + C_4 \cdot T^2$$

wobei die $C_i$ Konstanten sind.

Der Wobbe-Index W für das Restgasgemisch wird dann als lineare Funktion der Standardviskosität bestimmt, also

$$W = A \eta_{ref} + B, +C*M + D*\lambda_{ref}$$

wobei A und B, C und D Konstanten sind.

In einem Schritt 140 wird aus dem Dichtemesswert, sowie dem zugehörigen Druck- und Temperaturmesswerten die mittlere Molare Masse M(p, T, p) des Brenngasgemischs bestimmt.

Das Bestimmen der mittleren molaren Masse M des Gasgemisches umfasst in einer Ausgestaltung der Erfindung das Berechnen der mittleren molaren Masse als Funktion der Dichte, des Drucks und der Temperatur

$$M = f(\rho, T, p)$$

beispielsweise mittels eines Polynoms in p, p und T, also

$$M = \Sigma B_i \cdot T^{ti} \cdot \rho^{ri} \cdot p^{vi},$$

wobei i = 0 ... kein Index der Summanden ist, und die $B_i$ deren Koeffizienten sowie $t_i$, $r_i$ und $v_i$ ganzzahlige Exponenten sind.

Beispielsweise gilt:

$$M = B_0 + B_1 \cdot \rho \cdot T / p + B_2 \cdot \rho^2 \cdot T / p + B_3 \cdot \rho^2 / p + B_4 \cdot (\rho \cdot T / p)^2 + B_5 \cdot p,$$

Aus der mittleren molaren Masse M(p, T, ρ) und dem viskositätsabhängigen ersten Wert für den Wobbe-Index wird im nächsten Schritt 150 ein erster Wert $CV_\eta$ für den Brennwert des Brenngasgemischs ermittelt.

$$CV_\eta = C^* \, W \cdot (M / M_{Luft})^{1/2},$$

Weiterhin wird im nächsten Schritt 160 nur anhand der mittleren molaren Masse M(p, T, ρ), welche mit dem Brennwert korreliert, solange keine Inertgase vorhanden sind, ein zweiter Wert für den Brennwert des Brenngasgemischs ermittelt:

$$CV_M = D_0 + M \cdot D_1,$$

wobei die $D_i$ Konstanten sind.

Im nächsten Schritt 170 wird aus der Abweichung zwischen dem ersten Wert für den Brennwert des Brenngasgemischs und dem zweiten Wert für den Brennwert des Brenngasgemischs der Inertgasanteil %IG bestimmt:

$$\%IG = E \cdot (CV_M / CV_\eta - 1),$$

wobei E eine Konstante ist.

In einem daran anschließenden Schritt 180 wird auf Basis des Inertgasanteils %IG und der mittleren molaren Masse M des Brenngasgemischs die mittlere molare Masse $M_{RED}$(M, %IG) des um den Inertgasanteils reduzierten Brenngasgemischs ermittelt, welches im Wesentlichen aus Kohlenwasserstoffverbindungen besteht:

$$M_{RED} = (M - \%IG \cdot M_{IG}) / (1 - \%IG),$$

wobei $M_{IG}$ die mittlere molare Masse des Inertgases ist. Hierfür ergeben sich, je nach Erwartungswert für das Mischungsverhältnis zwischen Stickstoff und Kohlenstoffdioxid Werte zwischen 28 und 44, wobei Werte zwischen 30 und 34 insbesondere zwischen 30 und 32 besonders geeignet erscheinen.

Unter dieser Annahme kann in einem Folgeschritt 190 aus der mittleren molare Masse $M_{RED}$ des um den Inertgasanteil reduzierten Brenngasgemischs die Methanzahl $MZ_{RED}$($M_{RED}$) für ein Kohlenwasserstoffgemisch der mittleren molaren Masse $M_{RED}$ ermittelt werden, beispielsweise gemäß:

$$MZ_{RED} = A + B \cdot M_{RED} + C \cdot M_{RED}^2,$$

wobei A, B, C Konstanten sind.

In einem letzten Schritt 192 wird die Methanzahl $MZ_{RED}$ für das reine Kohlenwasserstoffgemisch noch um den Einfluss der Inertgase auf die Methanzahl korrigiert, um die Methanzahl $MZ_{total}$ für das strömende Brenngasgemisch zu erhalten, beispielsweise gemäß:

$$MZ_{total} = MZ_{RED} \cdot (A + B \cdot \%IG + C \cdot M_{RED}),$$

Wobei A, B, C Konstanten sind.

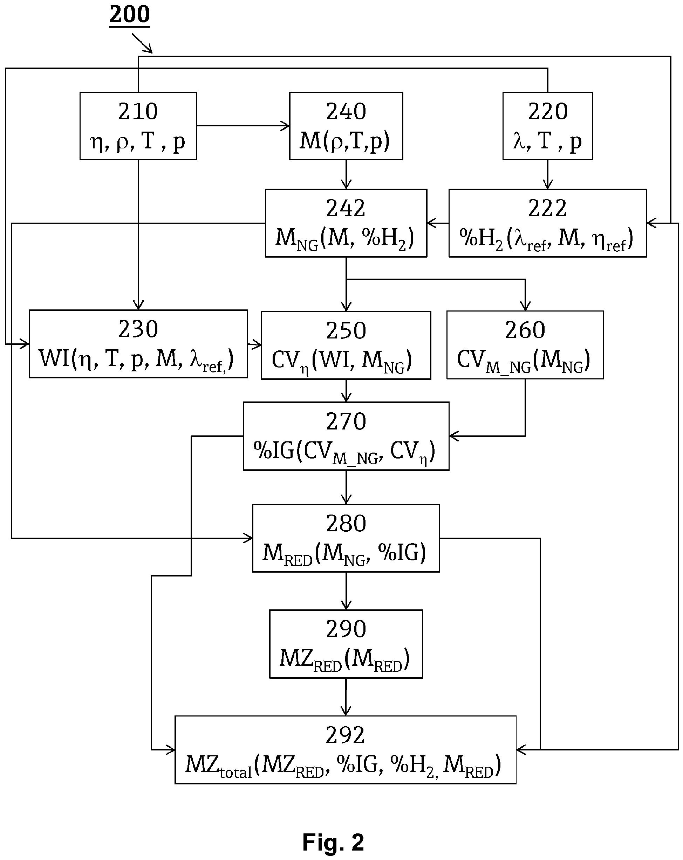

Das in Fig. 2 dargestellte zweite Ausführungsbeispiel des erfindungsgemäßen Verfahrens ist gegenüber dem ersten Ausführungsbeispiel modifiziert, um auch wasserstoffhaltige Brenngasgemische analysieren zu können. Das Verfahren 200 umfasst wie zuvor in einem Schritt 210 das Erfassen eines Viskositätsmesswerts η, eines Dichtemesswerts ρ, eines Temperaturmesswerts T und eines Druckmesswerts p des strömenden Brenngasgemischs. Die im Zusammenhang mit dem ersten Ausführungsbeispiel erläuterten Einzelheiten gelten hier entsprechend.

In einem zusätzlichen Schritt 220 wird noch ein Messwert für die Wärmeleitfähigkeit λ sowie zugehörige Druck p und Temperaturmesswerte T erfasst, um auf Basis dieses Wertetupels den Wasserstoffanteil $\%H_2$ in dem Brenngasgemisch zu ermitteln, wobei insbesondere zunächst aus dem Wärmeleitfähigeitsmesswert eine Referenzwärmeleitfähigkeit $\lambda_{ref}$ bei Referenzbedingungen berechnet wird, auf deren Basis dann der Wasserstoffanteil $\%H_2$ bestimmt wird.

Dem Bestimmen des Wärmeleitfähigkeitswerts $\lambda$ bei einem gegebenen Druck und einer gegebenen Temperatur folgt in einer Ausgestaltung der Erfindung eine Umrechnung in eine Standardwärmeleitfähigkeit $\lambda_{ref}$ bei Standardbedingungen, beispielsweise mit einem Polynom in $\lambda$, p und T, insbesondere

$$\lambda_{ref} = \Sigma\ A_i \cdot T^{ti} \cdot \lambda^{li} \cdot p^{vi},\ M,\ \eta_{ref}$$

wobei i= 0 ... kein Index der Summanden ist, und die $A_i$ deren Koeffizienten sowie $t_i$, $l_i$ und $v_i$ ganzzahlige Exponenten sind. Beispielsweise gilt:

$$\lambda_{ref} = A_0 + A_1 \cdot T + A_2 \cdot T^2 + A_3 \cdot p + A_4 \cdot \lambda + A_5 \cdot \lambda \cdot T. + A_6 * \eta_{ref}$$

Der Wasserstoffanteil wird dann bestimmt gemäß:

$$\%H_2 = A\ \lambda_{ref} + B + C*M + D* \eta_{ref}$$

In einem weiteren Schritt 230 wird auf Basis eines Viskositätsmesswerts, sei es der direkt gemessene Viskositätsmesswert oder ein daraus abgeleiteter Viskositätsmesswert bei Referenzbedingungen, ein erster Wert für den Wobbe-Index $WI(\eta, T, p)$ des Brenngasgemischs bestimmt, wobei dieser erste Wert für den Wobbe-Index unabhängig von der Dichte des Brenngasgemischs bestimmt wird.

In einem Schritt 240 wird aus dem Dichtemesswert, sowie dem zugehörigen Druck- und Temperaturmesswerten die mittlere molare Masse M(p, T, p) des Brenngasgemischs bestimmt. Aus der mittleren molaren Masse des Brenngasgemischs M(p, T, p) und dessen Wasserstoffanteil wird in einem nächsten Schritt die mittlere molare Masse $M_{NG}$ (M, $\%H_2$) eines um den Wasserstoffanteil bereinigten Gasgemischs berechnet.

Aus der mittleren molaren Masse $M_{NG}$ des um den Wasserstoffanteil bereinigten Gasgemischs und dem viskositätsabhängigen ersten Wert für den Wobbe-Index wird im nächsten Schritt 250 ein erster Wert $CV_\eta$ für den Brennwert des um den Wasserstoffanteil bereinigten Gasgemischs ermittelt.

Weiterhin wird im nächsten Schritt 260 nur anhand der mittleren molaren Masse $M_{NG}$ des um den Wasserstoffanteil bereinigten Gasgemischs ein zweiter Wert $C_{VM\_NG}(M_{NG})$ für den Brennwert des um den Wasserstoffanteil bereinigten Gasgemischs ermittelt.

Im nächsten Schritt 270 wird aus der Abweichung zwischen dem ersten Wert für den Brennwert des um den Wasserstoffanteil bereinigten Gasgemischs und dem zweiten Wert für den Brennwert des um den Wasserstoffanteil bereinigten Gasgemischs der Inertgasanteil %IG bestimmt.

In einem daran anschließenden Schritt 280 wird auf Basis des Inertgasanteils %IG und der mittleren molaren Masse $M_{NG}$ des um den Wasserstoffanteil bereinigten Gasgemischs die mittlere Molare Masse $M_{RED}(M_{NG}, \%IG)$ des um den Wasserstoffanteil und um den Inertgasanteil bereinigten Gasgemischs ermittelt, welches im Wesentlichen aus Kohlenwasserstoffverbindungen besteht. Unter dieser Annahme kann in einem Folgeschritt 290 aus der mittleren molare Masse $M_{RED}$ des um den Wasserstoffanteil und um den Inertgasanteil bereinigten Gasgemischs die Methanzahl $MZ_{RED}(M_{RED})$ für ein Kohlenwasserstoffgemisch der mittleren molaren Masse $M_{RED}$ ermittelt werden.

In einem letzten Schritt 292 wird die Methanzahl $MZ_{RED}$ für das reine Kohlenwasserstoffgemisch noch um den Einfluss der Inertgase und des Wasserstoffs auf die Methanzahl korrigiert, um die Methanzahl $MZ_{total}$ für das strömende Brenngasgemisch zu erhalten.

Das Ausführungsbeispiel einer erfindungsgemäßen Vorrichtung 300 zur Durchführung des erfindungsgemäßen Verfahrens umfasst eine von dem Gasgemisch durchströmbare Messzelle 310, in welcher hier nur schematisch dargestellte Sensorelemente, nämlich ein Cantileverschwinger 322 zum Bestimmen der Viskosität und der Dichte eines Gasgemischs in der Messzelle, ein Drucksensor 324, ein Temperatursensor 326, und ein Wärmeleitfähigkeitssensor 328 angeordnet sind. Die Sensorelemente sind vorzugsweise in MEMS-Technologie realisiert. Die einzelnen Sensorprinzipien sind dem Fachmann im vorliegenden Sachgebiet an sich bekannt und brauchen hier nicht näher erläutert zu werden. Die Vorrichtung umfasst weiterhin eine Betriebs- und Auswerteeinheit 320 zum Treiben der Sensorelemente, zum Auswerten von deren Signalen, um die primären Messgrößen, wie Viskosität, Druck, Temperatur, Wärmeleitfähigkeit und Dichte zu bestimmen, und zum Ermitteln der Methanzahl sowie von zum Ermitteln der Methanzahl erforderlichen Hilfsgrößen, beispielsweise der mittleren molaren Masse, des Wasserstoffanteils, des Wobbe Index und/oder des Brennwerts und/oder des Inertgasanteils eines die Messzelle 310 durchströmenden Gasgemischs. Die Betriebs- und Auswerteeinheit umfasst hierzu eine Recheneinheit, die kompakt oder modular aufgebaut sein kann, und insbesondere räumlich voneinander getrennte Module umfassen kann. Die Messzelle 310 ist insbesondere in einer Bypassanordnung an eine Gasleitung 330 angeschlossen, wobei ein Volumenstrom des Gasgemischs mittels einer Druckdifferenz über der Messzelle 310, beispielsweise aufgrund einer Blende bzw. einer Venturidüse in der Rohrleitung, oder mittels einer hier nicht dargestellten Pumpe durch die Messzelle 310 getrieben werden kann.

**Patentansprüche**

1. Verfahren (100; 200) zum Bestimmen der Methanzahl eines kohlenwasserstoffhaltigen Brenngasgemischs, welches insbesondere Erdgas oder Biogas aufweist, umfassend:

   Strömen Lassen des Brenngasgemischs durch eine Messanordnung;
   Bestimmen eines ersten Messwerts (110; 210) einer von der Viskosität des strömenden Brenngasgemischs abhängigen ersten Messgröße,
   Bestimmen eines zweiten Messwerts (110; 210) einer von der Dichte des strömenden Brenngasgemischs abhängigen zweiten Messgröße;
   Bestimmen eines zum ersten Messwert und zweiten Messwert zugehörigen Druckmesswerts (110; 210) des strömenden Brenngasgemischs, und
   Bestimmen (110; 210) eines zum ersten Messwert und zweiten Messwert zugehörigen Temperaturmesswerts des strömenden Brenngasgemischs; und
   Bestimmen der Methanzahl (190; 290) als Funktion des ersten Messwerts, des zweiten Messwerts, des Druckmesswerts und des Temperaturmesswert.

2. Verfahren nach Anspruch 1 wobei die erste Messgröße eine Dämpfung von Schwingungen eines dem Brenngasgemisch ausgesetzten Oszillators charakterisiert, oder einen Druckabfall über einer Drossel beschreibt.

3. Verfahren nach Anspruch 1 wobei die zweite Messgröße eine Eigenfrequenz eines dem Brenngasgemisch ausgesetzten Oszillators oder die Schallgeschwindigkeit des Brenngasgemischs ist.

4. Verfahren nach einem der vorhergehenden Ansprüche, weiterhin umfassend die Bestimmung eines die Wärmeleitfähigkeit des Brenngasgemischs charakterisierenden dritten Messwerts, wobei der Dritte Messwert ebenfalls in die Ermittlung der Methanzahl eingeht.

5. Verfahren nach einem der vorhergehenden Ansprüche , wobei das Verfahren umfasst:

   Bestimmen eines Viskositätsmesswerts des strömenden Brenngasgemischs;
   Bestimmen eines Dichte- oder Schallgeschwindigkeitsmesswerts des strömenden Brenngasgemischs;
   Bestimmen der dazugehörigen Temperatur- und Druckwerte;
   Bestimmen eines Werts für die mittlere molare Masse der in dem Brenngasgemisch enthaltenen Kohlenwasserstoffe als Funktion zumindest des Viskositätsmesswerts und des Dichte- oder Schallgeschwindigkeitsmesswerts sowie der dazugehörigen Temperatur- und Druckwerte;
   Bestimmen der Methanzahl als Funktion des Werts für die mittlere molaren Masse der in dem Brenngasgemisch enthaltenen Kohlenwasserstoffverbindungen.

6. Verfahren nach Anspruch 5,
   wobei das Bestimmen des Werts für die mittlere molare Masse der in dem Brenngasgemisch enthaltenen Kohlenwasserstoffverbindungen umfasst:

   Ermitteln eines Werts für die mittlere molare Masse des strömenden Brenngasgemischs in Abhängigkeit vom Dichte- oder Schallgeschwindigkeitsmesswert des strömenden Brenngasgemischs; und
   Ermitteln eines Anteils mindestens einer Fremdkomponente an dem Brenngasgemisch wobei die Fremdkomponente frei von Kohlenwasserstoffverbindungen ist;
   Ermitteln des Werts für die mittlere molare Masse der in dem Brenngasgemisch enthaltenen Kohlenwasserstoffverbindungen als die mittlere molare Masse des um die mindestens eine Fremdkomponente bereinigten Brenngasgemischs, in Abhängigkeit des ermittelten Werts für die mittlere molare Masse des strömenden Brenngasgemischs und des Anteils der mindestens einen Fremdkomponente an dem strömenden Brenngasgemisch.

7. Verfahren nach Anspruch 6, wobei die mindestens eine Fremdkomponente in dem Brenngasgemisch enthaltene Inertgase, insbesondere Kohlenstoffdioxyd und Stickstoff umfasst.

8. Verfahren nach Anspruch 7 wobei das Bestimmen des Inertgasanteils an dem strömenden Brenngasgemisch umfasst:

   Bestimmen eines ersten Werts einer den Energiegehalt des Brenngasgemischs charakterisierenden Größe in Abhängigkeit vom Viskositätsmesswert;
   Bestimmen eines zweiten Werts der den Energiegehalt des Brenngasgemischs charakterisierenden Größe in Abhängigkeit vom Dichte- oder Schallgeschwindigkeitsmesswert des strömenden Brenngasgemischs und unabhängig vom Viskositätsmesswert; und
   Bestimmen des Werts für den Inertgasanteil des strömenden Brenngasgemischs in Abhängigkeit von einer Abweichung zwischen dem ersten Wert und dem zweiten Wert der den Energiegehalt des Brenngasgemischs charakterisierenden Größe.

9. Verfahren nach Anspruch 8,
   wobei die den Energiegehalt charakterisierende zweite Größe der Wobbe Index oder der Brennwert des strömenden Brenngasgemischs ist.

10. Verfahren nach einem der Ansprüche 7 bis 9,
    wobei die mindestens eine Fremdkomponente den in dem Brenngasgemisch enthaltenen molekularen Wasserstoff umfasst, wobei der Anteil des molekularen Wasserstoffs auf Basis mittels einer Messung der Wärmeleitfähigkeit des Brenngasgemischs bestimmt wird.

11. Verfahren nach Anspruch 10,

    wobei der Anteil des molekularen Wasserstoffs an dem strömenden Brenngasgemisch bestimmt wird, und
    wobei die mittlere molare Masse des um den molekularen Wasserstoff bereinigten Brenngasgemischs in Abhängigkeit des ermittelten Werts für die mittlere molare Masse des strömenden Brenngasgemischs und des Anteils des molekularen Wasserstoffs an dem strömenden Brenngasgemisch bestimmt wird,
    wobei der Inertgasanteil an dem um den molekularen Wasserstoff bereinigten Brenngasgemisch bestimmt wird,
    wobei die mittlere molare Masse der in dem Brenngasgemisch enthaltenen Kohlenwasserstoffverbindungen in Abhängigkeit von der mittleren molaren Masse des um den molekularen Wasserstoff bereinigten Brenngasgemischs in Abhängigkeit des ermittelten Werts für die mittlere molare Masse des um den molekularen Wasserstoff bereinigten Brenngasgemischs und des Inertgasanteils an dem um den molekularen Wasserstoff bereinigten Brenngasgemisch bestimmt wird;
    wobei die Methanzahl des Brenngasgemischs enthaltenen Kohlenwasserstoffverbindungen anhand des ermittelten Werts für die mittlere molare Masse der in dem Brenngasgemisch enthaltenen Kohlenwasserstoffverbindungen bestimmt wird.

12. Verfahren nach Anspruch 11,
    wobei die Methanzahl des strömenden Brenngasgemischs auf Basis des ermittelten Werts für die Methanzahl an den in dem Brenngasgemisch enthaltenen Kohlenwasserstoffverbindungen bestimmt wird.

13. Vorrichtung (300) zum Bestimmen von zumindest der Methanzahl eines kohlenwasserstoffhaltigen Gasgemisches mit einem Verfahren nach einem der vorhergehenden Ansprüche, wobei die Vorrichtung umfasst:

    eine von dem Gasgemisch durchströmbare Messanordnung (310), mit einem Temperatursensor (324), einem Drucksensor (326) und
    einem vibronischen Sensor (322) zum Bestimmen eines Viskositätsmesswerts und ggf. eines Dichtemesswerts des strömenden Gasgemischs; und
    eine Auswertungseinheit (320) zum Berechnen von Eigenschaften des strömenden Gasgemischs, **dadurch gekennzeichnet, dass**
    die Auswertungseinheit (320) dazu eingerichtet ist, das Verfahren nach einem der Ansprüche 1 bis 12 durchzuführen.

14. Vorrichtung (300) nach Anspruch 13, wobei der vibronische Sensor (322) ein MEMS-Sensor ist, welcher mindestens ein durchströmbares, schwingfähiges Messrohr und/oder mindestens einen vom strömenden Gasgemisch umgebenen Oszillator, insbesondere in Form mindestens eines schwingfähigen Kragträgers oder einer schwingfähigen Stimmgabel, aufweist.

15. Vorrichtung (300) nach Anspruch 13 oder 14, weiterhin umfassend einen Wärmeleitfähigkeitssensor (328).

**Claims**

1. Procedure (100, 200) to determine the methane number of a fuel gas mixture containing hydrocarbons, said mixture particularly containing natural gas or biogas, wherein said procedure comprises the following steps:

   The fuel gas mixture is allowed to flow through a measuring arrangement;
   Determination of a first measured value (110, 210) of a first measured variable that depends on the viscosity of the flowing fuel gas mixture,
   Determination of a second measured value (110, 210) of a second measured variable that depends on the density of the flowing fuel gas mixture;
   Determination of a pressure measured value (110, 210) of the flowing fuel gas mixture, wherein said pressure measured value is associated with the first measured value and the second measured value, and
   Determination of a temperature measured value (110, 210) of the flowing fuel gas mixture, wherein said temperature measured value is associated with the first measured value and the second measured value; and
   Determination of the methane number (190, 290) as a function of the first measured value, of the second measured value, of the pressure measured value and of the temperature measured value.

2. Procedure as claimed in Claim 1, wherein the first measured variable characterizes a damping of oscillations of an oscillator exposed to the fuel gas mixture, or describes the pressure drop over a choke.

3. Procedure as claimed in Claim 1, wherein the second measured variable is a natural frequency of an oscillator exposed to the fuel gas mixture, or the speed of sound of the fuel gas mixture.

4. Procedure as claimed in one of the previous claims, further comprising the determination of a third measured value characterizing the thermal conductivity of the fuel gas mixture, wherein the third measured value is also included in the determination of the methane number.

5. Procedure as claimed in one of the previous claims, wherein said procedure comprises the following steps:

   Determination of a viscosity measured value of the flowing fuel gas mixture;
   Determination of a density measured value or the measured value of the speed of sound of the flowing fuel gas mixture;
   Determination of the associated temperature and pressure values;
   Determination of a value for the average molar mass of the hydrocarbons contained in the fuel gas mixture as a function at least of the viscosity measured value and density measured value or measured value of the speed of sound, as well as the associated temperature and pressure values;
   Determination of the methane number as a function of the value for the average molar mass of the hydrocarbon compounds contained in the fuel gas mixture.

6. Procedure as claimed in Claim 5,
   wherein the determination of the value for the average molar mass of the hydrocarbon compounds contained in the fuel gas mixture comprises the following steps:

   Determination of a value for the average molar mass of the flowing fuel gas mixture as a function of the measured value of density or of the speed of sound of the flowing fuel gas mixture; and
   Determination of a proportion of at least a foreign component in the fuel gas mixture, wherein said foreign component is free from hydrocarbon compounds;
   Determination of the value for the average molar mass of the hydrocarbon compounds contained in the fuel gas mixture as the average molar mass of the fuel gas mixture adjusted for the at least one foreign component, as a function of the value determined for the average molar mass of the flowing fuel gas mixture and of the proportion of the at least one foreign component in the flowing fuel gas mixture.

7. Procedure as claimed in Claim 6, wherein the at least one foreign component comprises inert gases contained in the fuel gas mixture, particularly carbon dioxide and nitrogen.

8. Procedure as claimed in Claim 7, wherein the determination of the proportion of inert gas in the flowing fuel gas mixture comprises the following steps:

Determination of a first value of a variable characterizing the energy contents of the fuel gas mixture as a function of the viscosity measured value;
Determination of a second value of the variable characterizing the energy contents of the fuel gas mixture as a function of the density measured value or the measured value of the speed of sound of the flowing fuel gas mixture and independently of the viscosity measured value; and
Determination of the value for the inert gas proportion of the flowing fuel gas mixture as a function of a deviation between the first value and the second value of the variable characterizing the energy contents of the fuel gas mixture.

9. Procedure as claimed in Claim 8,
wherein the second variable characterizing the energy contents is the Wobbe index or the calorific value of the flowing fuel gas mixture.

10. Procedure as claimed in one of the Claims 7 to 9,
wherein the at least one foreign component comprises the molecular hydrogen contained in the fuel gas mixture, wherein the proportion of the molecular hydrogen is determined on the basis of a measurement of the thermal conductivity of the fuel gas mixture.

11. Procedure as claimed in Claim 10,

wherein the proportion of the molecular hydrogen in the flowing fuel gas mixture is determined, and
wherein the average molar mass of the fuel gas mixture adjusted for the molecular hydrogen is determined as a function of the value determined for the average molar mass of the flowing fuel gas mixture and of the proportion of the molecular hydrogen in the flowing fuel gas mixture,
wherein the proportion of the inert gas in the fuel gas mixture adjusted for the molecular hydrogen is determined, wherein the average molar mass of the hydrocarbon compounds contained in the fuel gas mixture is determined as a function of the average molar mass of the fuel gas mixture adjusted for the molecular hydrogen as a function of the value determined for the average molar mass of the fuel gas mixture adjusted for the molecular hydrogen and of the proportion of the inert gas in the fuel gas mixture adjusted for the molecular hydrogen;
wherein the methane number of the hydrocarbon compounds contained in the fuel gas mixture is determined using the value determined for the average molar mass of the hydrocarbon compounds contained in the fuel gas mixture.

12. Procedure as claimed in Claim 11,
wherein the methane number of the flowing fuel gas mixture is determined on the basis of the value determined for the methane number of the hydrocarbon compounds contained in the fuel gas mixture.

13. Apparatus (300) designed to determine at least the methane number of a gas mixture containing hydrocarbons with a procedure as claimed in one of the previous claims, said apparatus comprising:

a measuring arrangement (310) through which the gas mixture can flow, with a temperature sensor (324), a pressure sensor (326) and
a vibronic sensor (322) designed to determine a viscosity measured value and,
where applicable, a density measured value of the flowing gas mixture; and
an evaluation unit (320) designed to calculate properties of the flowing gas mixture, **characterized in that** the evaluation unit (320) is designed to execute the procedure as claimed in one of the Claims 1 to 12.

14. Apparatus (300) as claimed in Claim 13, wherein the vibronic sensor (322) is a MEMS sensor which has at least a measuring tube which can vibrate and through which medium can flow, and/or at least an oscillator surrounded by the flowing gas mixture, particularly in the form of at least a cantilever beam which can vibrate or a tuning fork which can vibrate.

**15.** Apparatus (300) as claimed in Claim 13 or 14, further comprising a thermal conductivity sensor (328).

**Revendications**

**1.** Procédé (100, 200) destiné à la détermination de l'indice de méthane d'un mélange de gaz combustible contenant des hydrocarbures, lequel mélange contient notamment du gaz naturel ou du biogaz, lequel procédé comprend les étapes suivantes :

Mise en écoulement du mélange de gaz combustible à travers un dispositif de mesure ;
Détermination d'une première valeur mesurée (110, 210) d'une première grandeur de mesure dépendant de la viscosité du mélange de gaz combustible en écoulement,
Détermination d'une deuxième valeur mesurée (110, 210) d'une deuxième grandeur de mesure dépendant de la densité du mélange de gaz combustible en écoulement ;
Détermination d'une valeur mesurée de pression (110, 210) du mélange de gaz combustible en écoulement, laquelle valeur mesurée de pression est associée à la première valeur mesurée et à la deuxième valeur mesurée, et
Détermination d'une valeur mesurée de température (110, 210) du mélange de gaz combustible en écoulement, laquelle valeur mesurée de température est associée à la première valeur mesurée et à la deuxième valeur mesurée ; et
Détermination de l'indice de méthane (190, 290) en fonction de la première valeur mesurée, de la deuxième valeur mesurée, de la valeur de mesure de pression et de la valeur de mesure de température.

**2.** Procédé selon la revendication 1, pour lequel la première grandeur de mesure caractérise un amortissement des oscillations d'un oscillateur exposé au mélange de gaz combustible, ou décrit une chute de pression à travers un étranglement.

**3.** Procédé selon la revendication 1, pour lequel la deuxième grandeur de mesure est une fréquence propre d'un oscillateur exposé au mélange de gaz combustible, ou la vitesse du son du mélange de gaz combustible.

**4.** Procédé selon l'une des revendications précédentes, lequel procédé comprend en outre la détermination d'une troisième valeur mesurée caractérisant la conductivité thermique du mélange de gaz combustible, la troisième valeur mesurée intervenant également dans la détermination de l'indice de méthane.

**5.** Procédé selon l'une des revendications précédentes, lequel procédé comprend les étapes suivantes :

Détermination d'une valeur mesurée de viscosité du mélange de gaz combustible en écoulement ;
Détermination d'une valeur mesurée de densité ou de vitesse du son du mélange de gaz combustible en écoulement ;
Détermination des valeurs de température et de pression correspondantes ;
Détermination d'une valeur pour la masse molaire moyenne des hydrocarbures contenus dans le mélange de gaz combustible en fonction au moins de la valeur mesurée de viscosité et de la valeur mesurée de densité ou de vitesse du son, ainsi que des valeurs de température et de pression correspondantes ;
Détermination de l'indice de méthane en fonction de la valeur de la masse molaire moyenne des composés hydrocarbonés contenus dans le mélange de gaz combustible.

**6.** Procédé selon la revendication 5,
pour lequel la détermination de la valeur de la masse molaire moyenne des composés hydrocarbonés contenus dans le mélange de gaz combustible comprend les étapes suivantes :

Détermination d'une valeur de la masse molaire moyenne du mélange de gaz combustible en écoulement en fonction de la valeur mesurée de densité ou de vitesse du son du mélange de gaz combustible en écoulement ; et
Détermination d'une proportion d'au moins un composant étranger dans le mélange de gaz combustible, le composant étranger étant exempt de composés hydrocarbonés ;
Détermination de la valeur pour la masse molaire moyenne des composés d'hydrocarbures contenus dans le mélange de gaz combustible en tant que masse molaire moyenne du mélange de gaz combustible épuré de l'au moins un composant étranger, en fonction de la valeur déterminée pour la masse molaire moyenne du mélange de gaz combustible en écoulement et de la proportion de l'au moins un composant étranger dans le mélange de gaz combustible en écoulement.

7. Procédé selon la revendication 6, pour lequel ledit au moins un composant étranger comprend des gaz inertes contenus dans le mélange de gaz combustible, notamment du dioxyde de carbone et de l'azote.

8. Procédé selon la revendication 7, pour lequel la détermination de la proportion de gaz inerte dans le mélange de gaz combustible en écoulement comprend les étapes suivantes :

Détermination d'une première valeur d'une grandeur caractérisant le contenu énergétique du mélange de gaz combustible en fonction de la valeur mesurée de viscosité ;
Détermination d'une deuxième valeur de la grandeur caractérisant le contenu énergétique du mélange de gaz combustible en fonction de la valeur mesurée de densité ou de la vitesse du son du mélange de gaz combustible en écoulement et indépendamment de la valeur mesurée de viscosité ; et
Détermination de la valeur de la fraction de gaz inerte du mélange de gaz combustible en écoulement en fonction d'un écart entre la première valeur et la deuxième valeur de la grandeur caractérisant le contenu énergétique du mélange de gaz combustible.

9. Procédé selon la revendication 8,
pour lequel la deuxième grandeur caractérisant le contenu énergétique est l'indice de Wobbe ou le pouvoir calorifique du mélange de gaz combustible en écoulement.

10. Procédé selon l'une des revendications 7 à 9,
pour lequel ledit au moins un composant étranger comprend l'hydrogène moléculaire contenu dans le mélange de gaz combustible, la proportion d'hydrogène moléculaire étant déterminée sur la base d'une mesure de la conductivité thermique du mélange de gaz combustible.

11. Procédé selon la revendication 10,

pour lequel la proportion d'hydrogène moléculaire dans le mélange de gaz combustible en écoulement est déterminée, et
pour lequel la masse molaire moyenne du mélange de gaz combustible épuré de l'hydrogène moléculaire est déterminée en fonction de la valeur déterminée pour la masse molaire moyenne du mélange de gaz combustible en écoulement et de la proportion d'hydrogène moléculaire dans le mélange de gaz combustible en écoulement,
pour lequel la proportion de gaz inerte dans le mélange de gaz combustible épuré de l'hydrogène moléculaire est déterminée,
pour lequel la masse molaire moyenne des composés hydrocarbonés contenus dans le mélange de gaz combustible est déterminée en fonction de la masse molaire moyenne du mélange de gaz combustible épuré de l'hydrogène moléculaire en fonction de la valeur déterminée pour la masse molaire moyenne du mélange de gaz combustible épuré de l'hydrogène moléculaire et de la proportion de gaz inerte dans le mélange de gaz combustible épuré de l'hydrogène moléculaire ;
pour lequel le nombre de composés hydrocarbonés contenus dans le mélange de gaz combustible est déterminé à l'aide de la valeur déterminée pour la masse molaire moyenne des composés hydrocarbonés contenus dans le mélange de gaz combustible.

12. Procédé selon la revendication 11,
pour lequel l'indice de méthane du mélange de gaz combustible en écoulement est déterminé sur la base de la valeur déterminée pour l'indice de méthane des composés hydrocarbonés contenus dans le mélange de gaz combustible.

13. Dispositif (300) destiné à la détermination d'au moins l'indice de méthane d'un mélange gazeux hydrocarboné à l'aide d'un procédé selon l'une des revendications précédentes, le dispositif comprenant :

un dispositif de mesure (310) pouvant être traversé par le mélange gazeux, comprenant
un capteur de température (324),
un capteur de pression (326) et
un capteur vibronique (322) destiné à la détermination d'une valeur mesurée de viscosité et, le cas échéant, d'une valeur mesurée de densité du mélange gazeux en écoulement ; et
une unité d'évaluation (320) destinée à calculer des propriétés du mélange gazeux en écoulement, **caractérisé**

**en ce que** l'unité d'évaluation (320) est conçue pour mettre en oeuvre le procédé selon l'une des revendications 1 à 12.

14. Dispositif (300) selon la revendication 13, pour lequel le capteur vibronique (322) est un capteur MEMS qui comporte au moins un tube de mesure apte à vibrer pouvant être traversé par un écoulement, et/ou au moins un oscillateur entouré par le mélange gazeux en écoulement, notamment sous la forme d'au moins une poutre cantilever apte à vibrer ou d'un diapason apte à vibrer.

15. Dispositif (300) selon la revendication 13 ou 14, comprenant en outre un capteur de conductivité thermique (328).

**100**

| 110 | 140 |
| :-: | :-: |
| $\eta, \rho, T, p$ | $M(\rho, T, p)$ |

| 130 | 150 | 160 |
| :-: | :-: | :-: |
| $WI(\eta, T, p, M)$ | $CV_\eta(WI, M)$ | $CV_M(M)$ |

| 170 |
| :-: |
| $\%IG(CV_M, CV_\eta)$ |

| 180 |
| :-: |
| $M_{RED}(M, \%IG)$ |

| 190 |
| :-: |
| $MZ_{RED}(M_{RED})$ |

| 192 |
| :-: |
| $MZ_{total}(MZ_{RED}, \%IG, M_{RED})$ |

**Fig. 1**

**200**

| 210 $\eta, \rho, T, p$ | → | 240 $M(\rho, T, p)$ | | 220 $\lambda, T, p$ |

| | | 242 $M_{NG}(M, \%H_2)$ | ← | 222 $\%H_2(\lambda_{ref}, M, \eta_{ref})$ |

| 230 $WI(\eta, T, p, M, \lambda_{ref,})$ | 250 $CV_\eta(WI, M_{NG})$ | 260 $CV_{M\_NG}(M_{NG})$ |

| 270 $\%IG(CV_{M\_NG}, CV_\eta)$ |

| 280 $M_{RED}(M_{NG}, \%IG)$ |

| 290 $MZ_{RED}(M_{RED})$ |

| 292 $MZ_{total}(MZ_{RED}, \%IG, \%H_2, M_{RED})$ |

**Fig. 2**

**300**

320

322
324
326
328

310

330

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102014115566 A1
- DE 102014119212 A1
- WO 2015075278 A1
- EP 2574918 A1
- DE 102015117468
- DE 102016121226
- DE 102016124910